# EUROPEAN PATENT SPECIFICATION

(11) **EP 4 366 659 B1**
(45) Date of publication and mention of the grant of the patent: **25.11.2026**
(21) Application number: 21758198.2
(22) Date of filing: 08.07.2021
(51) Int. Cl.: A61F 2/07, A61F 2/06

(54) **PROSTHESIS WITH INTEGRATED THERAPEUTIC DELIVERY SYSTEM**
PROTHESE MIT INTEGRIERTEM THERAPEUTISCHEM ABGABESYSTEM
PROTHÈSE AVEC SYSTÈME D'ADMINISTRATION THÉRAPEUTIQUE INTÉGRÉ

(43) Date of publication of application: 15.05.2024
(73) Proprietor: Bard Peripheral Vascular, Inc., Franklin Lakes, NJ 07417 (US)
(72) Inventor: SOLOMON, Clint, Gillbert, AZ 85234 (US); SANCHEZ GARCIA, Edgar, Tempe, AZ 85284 (US)
(74) Representative: Hoffmann Eitle
(86) International application number: PCT/US2021/040930
(87) International publication number: WO 2023/282903

(56) References cited:
- US-A- 5 411 550
- US-A1- 2008 234 809
- US-A1- 2014 303 716
- US-B1- 6 355 063

## Description

### BACKGROUND

US 6,355,063 B1 discloses an improved ePTFE-based delivery graft intended to dispense a bioactive agent such as a drug into the blood stream. A hollow tubing is infused with the agent from a source such as a drug delivery pump mechanism. The spiral hollow tubing is wrapped in a helical fashion around, or otherwise brought into contact with an outer wall of a porous interstices of the graft wall. Thus, the bioactive agent is conducted by the hollow tubing from a source to the outer surface of an ePTFE graft where it is released to diffuse into the graft to influence biological processes along both the inner and outer surfaces of the graft.

US 2008/0234809 A1 discloses a stent graft system with injection tube.

### SUMMARY

Briefly summarized, embodiments disclosed herein are directed to an implantable prosthesis, or graft, having an integrated therapeutic delivery system. Providing medications, drugs, or similar therapeutic agents directly into the blood stream can increase the efficacy of the treatments. Further, when placing grafts, stents, or similar intravascular prostheses, drugs may be required to prevent rejection of the prosthesis, restenosis, or similar unwanted side effects. Administering such drugs systemically can reduce the efficacy of the drugs at the target site, or affect areas other than the target site.

Vascular prostheses have been developed that include drug eluting structures. These structures can provide therapeutic agents directly to the target site, increasing the efficacy of the treatment and extending the lifespan of the prosthesis. However, even these slow-release, drug-eluting structures still only have a finite efficacy time. Embodiments, disclosed herein are directed to an implantable graft having an integrated therapeutic delivery system that can be replenished indefinitely to increase the lifespan of the prosthesis.

The invention relates to a drug delivery graft as defined in independent claim 1.

Disclosed herein is a drug delivery graft including, a graft body defining a graft lumen extending between a first end and a second end, an implantable access port, a delivery line coupled to the graft body, and defining a delivery line lumen in fluid communication with the implantable access port, and a plurality of channels extending through a wall of the graft body and communicating between the graft lumen and the delivery line lumen.

In some embodiments, a portion of the delivery line is coupled to an outer surface of the graft body or embedded within the wall of the graft body, or partially embedded within the wall of the graft body. In some embodiments, the delivery line extends helically about the graft body. In some embodiments, the delivery line extends laterally, longitudinally, or radially along the graft body. In some embodiments, the plurality of channels are spaced equidistant throughout the graft body. In some embodiments, the plurality of channels have an equal lumen diameter. In some embodiments, a distance between a first channel and a second channel of the plurality of channels disposed proximate the first end, is larger than a distance between a third channel and a fourth channel of the plurality of channels disposed proximate the second end.

In some embodiments, a diameter of the first channel of the plurality of channels disposed proximate the first end is smaller than a diameter of the fourth channel of the plurality of channels disposed proximate the second end. In some embodiments, the implantable access port is coupled to the delivery line proximate the second end. In some embodiments, the diameter of the delivery line proximate the second end is larger than the diameter of the delivery line proximate the first end. In some embodiments, one or both of the graft body and the delivery line is formed of an impermeable material. In some embodiments, one or both of the first end or the second end are trimmable from an original length to a second selected length, shorter than the original length.

Also disclosed, but not encompassed by the scope of the claims, is a method of infusing a drug including, accessing a subcutaneous access port with an access needle, inserting a predetermined amount of therapeutic fluid into the port, flowing the therapeutic fluid through a delivery line, the delivery line coupled to a surface of a graft body, and flowing the therapeutic fluid through a plurality of channels into a lumen of the graft body.

A portion of the delivery line may be coupled to an outer surface of the graft body or embedded within the wall of the graft body, or partially embedded within the wall of the graft body. The delivery line may extend helically about the graft body. The delivery line may extends laterally, longitudinally, or radially along the graft body. The plurality of channels may be spaced equidistant throughout the graft body. The plurality of channels may have an equal lumen diameter. One or both of the graft body and the delivery line may be formed of an impermeable material.

### DRAWINGS

A more particular description of the present disclosure will be rendered by reference to specific embodiments thereof that are illustrated in the appended drawings. It is appreciated that these drawings depict only typical embodiments of the invention and are therefore not to be considered limiting of its scope. Example embodiments of the invention will be described and explained with additional specificity and detail through the use of the accompanying drawings in which:
FIG. 1 shows a perspective view of a graft system, in accordance with embodiments disclosed herein.
FIGS. 2A-2B show close up detail of a cross-sectional view of a graft system, in accordance with embodiments disclosed herein.
FIGS. 3A-3E show schematic views of various graft systems, in accordance with embodiments disclosed herein.
FIGS. 4A-4B shows a schematic view of a graft system, in accordance with embodiments disclosed herein.

### DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

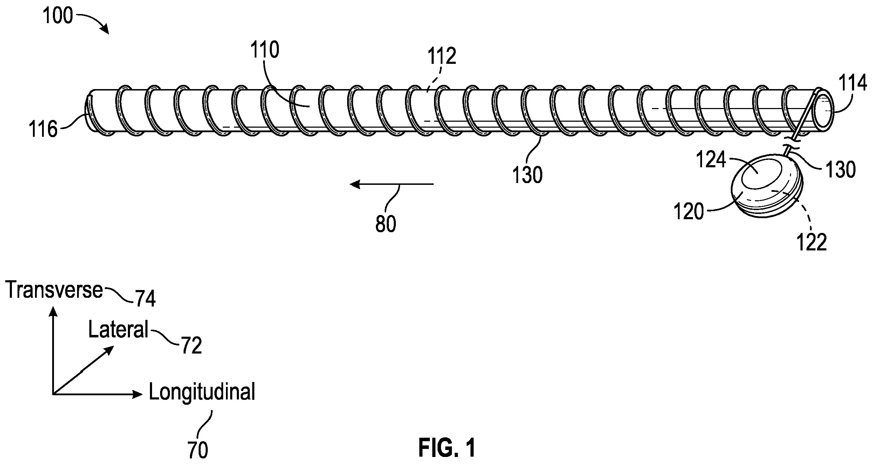

To assist in the description of embodiments described herein, as shown in FIG. 1 a longitudinal axis 70 extends substantially parallel to an axial length of the graft 100. A lateral axis 72 extends normal to the longitudinal axis, and a transverse axis 74 extends normal to both the longitudinal and lateral axes.

Unless defined otherwise, all technical and scientific terms used herein have the same meaning as commonly understood by those of ordinary skill in the art.

FIG. 1 shows an embodiment of an implantable prosthesis, or graft, having an integrated therapeutic delivery system ("graft system") 100. The graft system 100 generally includes a graft body 110, a port 120, and a delivery line 130 in fluid communication with the port 120 and coupled to the graft body 110. The delivery line 130 can be configured to deliver therapeutic agents, drugs, anticoagulants, heparin or the like, from the port 120 to a lumen 112 of the graft body 110. The graft body 110 can define a substantially cylindrical shape having a circular cross-section. However it will be appreciated that other cross-sectional shapes, e.g. elliptical, etc., are also contemplated. The graft body 110 can define a lumen 112 extending therethrough between a first end 114 of the graft body 110 and a second end 116 of the graft body 110. As shown, a blood flow 80 can flow through the graft body lumen 112 from the first end 114 to the second end 116.

In an embodiment, the graft body 110 can be disposed within a vasculature of a patient, such as arteries, veins, capillaries, or the like. However it will be appreciated that embodiments disclosed herein can be used within any tubular structure within the patient, including but not limited to, the lymph system, uro-excretory system, or the like. In an embodiment, the graft system 100 can be used with various other vascular medical devices, (e.g. stents, or the like) either disposed within the graft lumen 112, or disposed abluminally on an outer surface of the graft body 110. The port 120 can be disposed subcutaneously, adjacent a skin surface with a portion of the delivery line 130 providing fluid communication between the port 120 and the graft body 110.

In an embodiment, the graft body 110 can be formed of an impervious material, such as polypropylene (PP), non-porous polytetrafluoroethylene (PTFE), fluoroethylene polymer (FEP), or similar implantable polymer or plastic Advantageously, the impervious material can maintain the therapeutic agent within the lumen 112 of the graft body 110 to allow the therapeutic agent to fully mix with the blood stream. Further, the impervious graft body 110 can mitigate reabsorption of the therapeutic agent proximate the locus of introduction. As such, the graft body 110 can promote a more uniform dispersion of the therapeutic agent throughout the graft body 110 and/or downstream thereof.

As will be appreciated the longitudinal length of the graft body 110 can vary depending on the requirements of the treatment. In an embodiment, one of the first end 114 or the second end 116 can be trimmable from an original length to a second, selected length. In an embodiment, the graft body 110 can be formed of a porous or semi-porous material, such as expanded polytetrafluoroethylene (ePTFE), or similar porous implantable polymer or plastic. Further details and embodiments of which can be found in U.S. Patent No. 6,355,063.

In an embodiment, the port 120 can include a reservoir 122 having a needle penetrable septum 124 disposed thereover. The septum 124 can provide percutaneous access to the reservoir 122 by an access needle. For example, an access needle (e.g. Huber needle, non-coring needle, or the like) can penetrate the skin surface and underlying tissues and penetrate the septum 124 to access the reservoir 122. The access needle can define a lumen to provide fluid communication with the reservoir. In an embodiment, the port 120 can be coupled to the delivery line 130 by way of an interference fit, press-fit, snap-fit engagement, or can be coupled to a stem of the port 120 using a cathlock or similar mechanism. In an embodiment, the delivery line 130 can be formed integrally with the port 120 or coupled thereto by adhesive, bonding, welding, combinations thereof, or the like. In an embodiment, the port 120 can provide fluid communication between the reservoir 122 and a lumen 132 of the delivery line 130. It will be appreciated that the port 120 is an exemplary access device and various subcutaneous or supra-cutaneous access devices can be used with the graft system 100, for example the devices disclosed in U.S. Patent No. 8,998,860; U.S. Patent No. 9,642,986; U.S. Patent No. 10,307,581; U.S. Patent Publication No. 2019/0232035; and WO 2020/028847.

The delivery line 130 can be a tubular structure defining a lumen 132 in fluid communication with the port 120. The delivery line 130 can extend from the port 120 to the graft body 110 and can be coupled with an outer surface of the graft body 110 by adhesive, bonding, welding, or can be formed integrally therewith. The delivery line 130 can extend over at least a portion of an outer surface of the graft body 110. As shown in FIG. 1, the delivery line 130 can extend helically about the graft body 110. However it will be appreciated that other arrangements of one or more delivery lines 130 are also contemplated, as described in more detail herein.

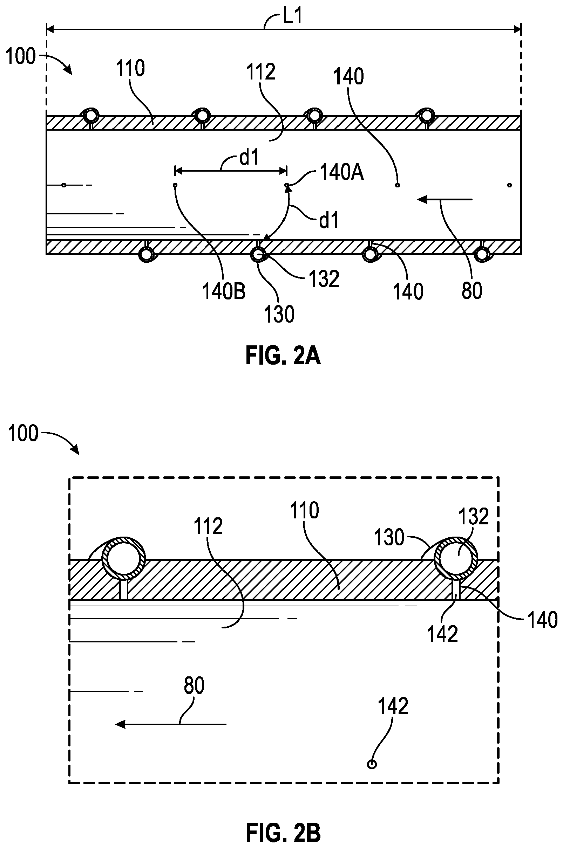

In an embodiment, the delivery line 130 can extend from the port 120 to a first end 114 of the graft body 110 and then to a second end 116 of the graft body 110. However, it will be appreciated that the delivery line 130 can extend from the port 120 to the graft body 110 to either the first end 114, the second end 116, or to a point disposed therebetween, as described in more detail herein. In an embodiment, the delivery line 130, or a portion thereof, can extend over an outer surface of the graft body 110. In an embodiment, the delivery line 130, or a portion thereof, can be embedded within the wall of the graft body 110, i.e. extending through a wall of the graft body 110. In an embodiment, as shown in FIGS. 2A-2B, the delivery line 130, or a portion thereof, can be partially embedded within the wall of the graft body 110, i.e. a portion of the delivery line 130 extends through the wall of the graft body 110 while an opposite portion protrudes from an outer surface of the graft body 110.

As shown in FIGS. 2A-2B, in an embodiment, the graft system 100 further includes one or more channels 140 extending through a wall of the graft body 110, substantially perpendicular to the longitudinal axis. However, it will be appreciated that other angles, relative to the longitudinal axis are also contemplated. The channels 140 define a channel lumen 142 providing fluid communication between a lumen 112 of the graft body 110 and a lumen 132 of the delivery line 130. In an embodiment, the channels 140 can be dispersed evenly, i.e. equidistant, throughout the lumen 112. For example, a distance (*d1*) over a surface of the lumen 112 can be equal between a first channel 140A and a second channel 140B. Advantageously, the distance (*d1*) disposed between one or more channels 140 can be modified to vary the concentration of therapeutic agents released into the graft lumen 112. For example, a relatively shorter distance (*d1*) can provide a greater number of channels 140 for a given longitudinal length (*L1*) of the graft body 110 which provides a higher rate of infusion and higher concentration of the therapeutic agent. Similarly, a relatively longer distance (*d1*) can provide a fewer number of channels 140 for a given longitudinal length (*L1*) of the graft body 110 which provides a lower rate of infusion and lower concentration of the therapeutic agent.

In an embodiment, a diameter of the channel lumen 142 can be modified to vary the concentration of therapeutic agents released into the graft lumen 112. For example, a relatively larger diameter of the channel lumen 142 can provide a higher rate of infusion and higher concentration of the therapeutic agent. Similarly, a relatively smaller diameter of the channel lumen 142 can provide a lower rate of infusion and a lower concentration of the therapeutic agent.

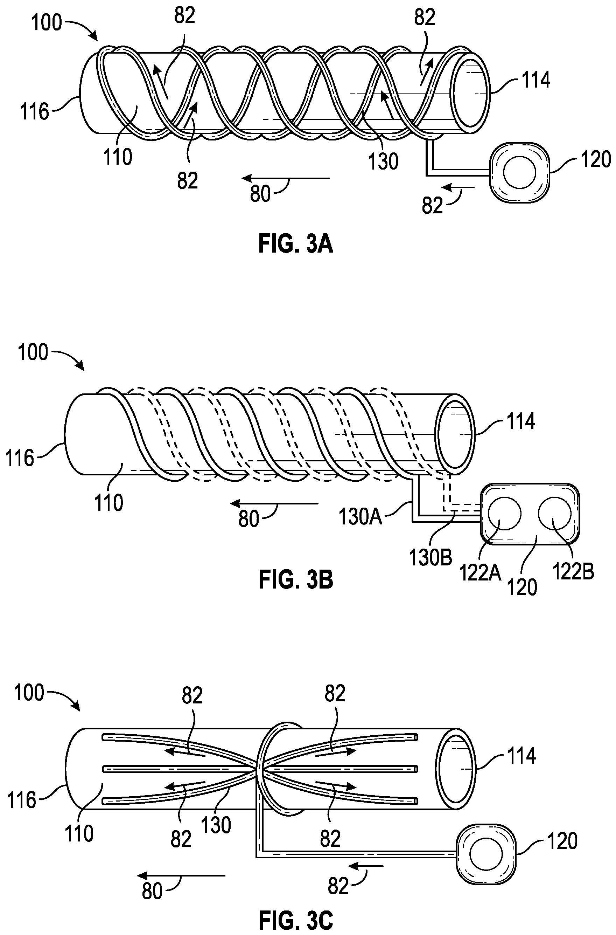

FIGS. 3A-3E show various configurations of delivery line 130 that can extend over the graft body 110. For example, the delivery line 130 can extend over the graft body 110 in a helical pattern (FIG. 1) or in a double helix (FIG. 3A, 3B). FIG. 3A shows a double helix extending in opposite directions about the graft body 110. For example, the delivery line 130 can extend in a first direction from the first end 114 to the second end 116 and can extend in a second direction about the graft body 110 from the second end 116 to the first end 114. As such, a flow 82 of therapeutic agents can flow from the port 120 to the first end 114, to the second end 116 and then back towards the first end 114. Advantageously, the double helix extending in opposite directions can allow the delivery line 130 to couple to the graft body 110 at for example a first end 114, and provide uniform dispersion of therapeutic fluids across the length of the graft body 110 rather than focused at a particular locus.

In an embodiment, as shown in FIG. 3B, the graft system 100 can include two or more delivery lines 130, for example a first delivery line 130A in fluid communication with a first reservoir 122A of the port 120, and a second delivery line 130B in fluid communication with a second reservoir 122B. Advantageously, two different therapeutic agents can be introduced simultaneously. This can be of particular importance were different flow rates or concentrations are required. In an embodiment, as shown in FIG. 3C, the delivery line 130 can extend from the port 120 to a point on the graft body 110, e.g. a mid-point and can extend in a radial or dendritic pattern over the surface of the graft body 110. As such, the delivery line 130 can be coupled with the graft body 110 at a central position and a flow of therapeutic agents 82 spread over the graft body 110 providing a shortest possible route to all portions of the graft body 110. Advantageously, the therapeutic agents can be delivered expediently to all portions of the graft body 110.

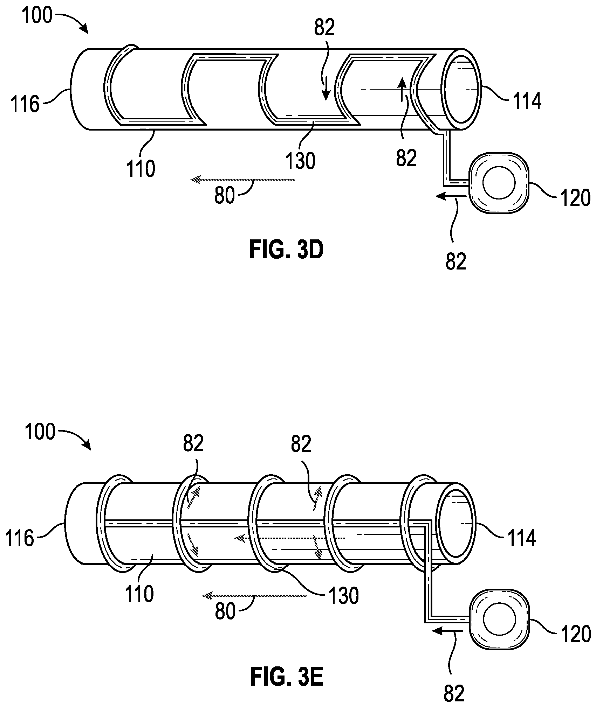

In an embodiment, as shown in FIGS. 3D-3E the delivery line 130 can extend longitudinally or laterally about the graft body 110 and can be arranged in series, where a first longitudinal/lateral portion is coupled to an adjacent longitudinal/lateral portion. Advantageously, the therapeutic agents can be delivered evenly throughout the graft body 110 In an embodiment, as shown in FIG. 3E the delivery line 130 can be connected in parallel where one or more longitudinal/lateral portions are coupled to a manifold extending therebetween. Further details of which can also be found in U.S. Patent No. 6,355,063. Advantageously, the configuration of the delivery line 130 on the graft body 110 can ensure a uniform and expeditious dissemination of the therapeutic agent along the length of the graft body 110.

In an embodiment, the graft body 110 can be formed of an impervious material. As such, the therapeutic agents can be released into the graft lumen 112 through one or more channel lumen 142. Advantageously, the number and diameter of channels 142 can be modified to provide an accurate, predetermined, rate of infusion of therapeutic agent into the lumen 112 of the graft body 110. Further, the impervious delivery line 130 and/or graft body 110 can ensure uniform dissemination of the therapeutic agent along the length of the graft body 110.

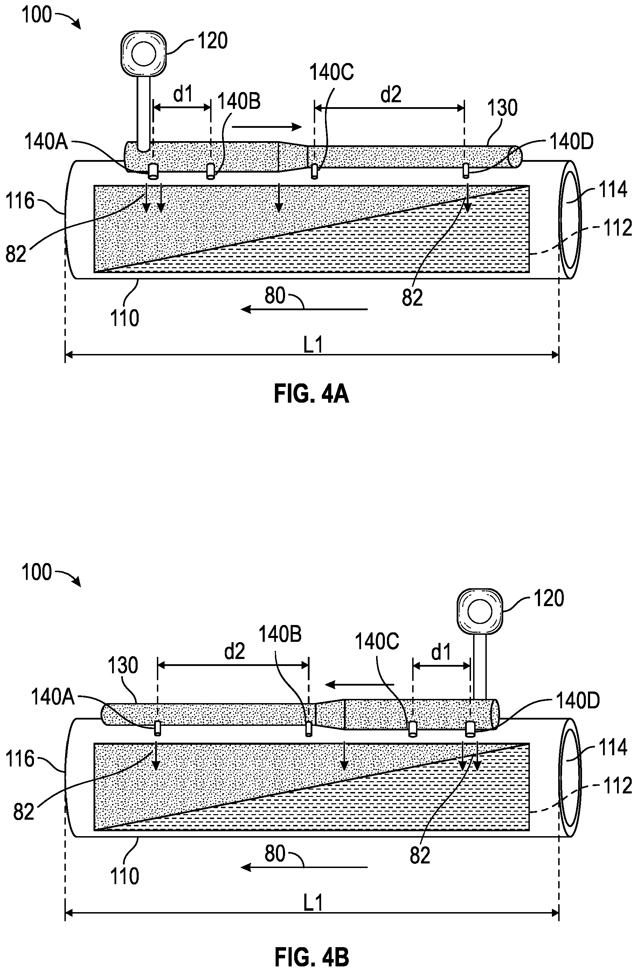

In an embodiment, as shown in FIGS. 4A-4B, the distance (*d1*) between the channels 140 and/or the diameter of the channel lumen 142 can be varied over the longitudinal length (*L1*) of the graft body 110. In an embodiment, the distance (*d1*) and/or the diameter of the channel lumen 142 can be varied regularly or irregularly over the longitudinal length (*L1*) of the graft body 110. In an embodiment, a diameter of the delivery line 130 can be uniform or can be varied over the length (*L1*) of the graft 110. As such, one or more of the channel 140 density, total number of channels 140, diameter of the channels 142, or diameter of the delivery line 130 can be modified over the length (*L1*) of the graft body 110 to provide varying rates of infusion of the therapeutic agent, or varying flow rates 82 of therapeutic agent. Advantageously, the different flow rates 82 over the over the length (*L1*) of the graft body 110 can offset different concentrations of the therapeutic agent within the lumen 112 and/or the direction of blood flow 80 to provide a uniform dispersion of therapeutic agent.

For example, as shown in FIG. 4A, a blood flow 80 can flow through the graft lumen 112 from the first end 114 to the second end 116. The port 120 can be coupled to the delivery line 130 proximate the second end 116 and a therapeutic agent can flow through the delivery line 130 from the second end 116 to the first end 114, i.e. counter to the blood flow 80. In an embodiment, a distance between the channels 140 (e.g. between a first channel 140A and a second channel 140B) proximate to the second end 116 (i.e. a first distance (*d1*)) can be less than a distance between the channels 140 proximate to the first end 114 (i.e. a second distance (*d2*)). In an embodiment, the second distance (*d2*) can be between 101% and 200% that of the first distance (*d1*)*.* However it will be appreciated that smaller or larger ratios of distances between channels 140 are also contemplated. In an embodiment, a diameter of the channel lumen 142 proximate to the second end 116 can be larger than a diameter of the channel lumen 142 proximate to the first end 114.

As such, a counter current infusion rate can be predetermined across the length (*L1*) of the graft body 110. Proximate the first end 114, upstream of the blood flow 80 a concentration of therapeutic agents within the graft lumen 112 is relatively low. As such, a lower infusion rate (i.e. greater spacing of channels 140, relatively smaller channel lumen 142 diameter, or smaller delivery line lumen diameter) is required to achieve an infusion rate. As the blood flows downstream through the graft lumen 112 towards second end 116, the concentration of therapeutics increases and, as such, a larger flow rate is required to infuse the therapeutics at the same rate.

Alternatively, as shown in FIG. 4B, a greater infusion rate may be required at an upstream position within the graft lumen 112, i.e. proximate the first end 114. As such, in an embodiment, the delivery line 130 may extend from the port 120 to the first end 114 of the graft body 110. In an embodiment, a diameter of the delivery line 130 proximate the first end 114 may be larger than a diameter of the delivery line 130 proximate the second end 116. In an embodiment, a density of channels 140 may be higher proximate the first end 114 (i.e. a distance (*d1*) between the third channel 140C and the fourth channel 140D, proximate the first end 114 may be shorter than a second distance (*d2*) between the first channel 140A and the second channel 140B, proximate the second end 116.) In an embodiment, the diameter of the channel lumen 142 proximate the first end 114 can be larger than a diameter of the channel lumen 142 proximate the second end 116.

While some particular embodiments have been disclosed herein, and while the particular embodiments have been disclosed in some detail, it is not the intention for the particular embodiments to limit the scope of the concepts provided herein. Accordingly, departures may be made from the particular embodiments disclosed herein without departing from the scope of the invention which is defined by the appended claims.

## Claims

1. A drug delivery graft (199), comprising:
a graft body (110) defining a graft lumen (112) extending between a first end (114) and a second end (116);
an implantable access port (120);
a delivery line (130) coupled to the graft body (110) and defining a delivery line lumen (132) in fluid communication with the implantable access port (120); the drug delivery graft being **characterized in that** it comprises
a plurality of channels (140) extending through a wall of the graft body (110) and communicating between the graft lumen (112) and the delivery line lumen (132), wherein the channels (140) define a channel lumen providing fluid communication between the graft lumen (112) and the delivery line lumen (132).

2. The drug delivery graft according to claim 1, wherein a portion of the delivery line (130) is coupled to an outer surface of the graft body (110), or embedded within the wall of the graft body (110), or partially embedded within the wall of the graft body (110).

3. The drug delivery graft according to any one of claims 1-2, wherein the delivery line (130) extends helically about the graft body (110).

4. The drug delivery graft according to any one of claims 1-2, wherein the delivery line (130) extends laterally about the graft body (110) or longitudinally along the graft body (110), or extends from a point on the graft body and extends in a radial pattern over the surface of the graft body (110).

5. The drug delivery graft according to any one of claims 1-4, wherein the plurality of channels (140) are spaced equidistant throughout the graft body (110).

6. The drug delivery graft according to any one of claims 1-5, wherein the plurality of channels (140) have an equal lumen diameter.

7. The drug delivery graft according to any one of claims 1-4, wherein a distance between a first channel (140A) and a second channel (140B) of the plurality of channels disposed proximate the first end (114), is larger than a distance between a third channel (140C) and a fourth channel (140D) of the plurality of channels disposed proximate the second end (116).

8. The drug delivery graft according to claim 7, wherein a diameter of the first channel (140A) of the plurality of channels disposed proximate the first end (114) is smaller than a diameter of the fourth channel (140D) of the plurality of channels disposed proximate the second end (116).

9. The drug delivery graft according to any one of claims 1-8, wherein the implantable access port (120) is coupled to the delivery line (130) proximate the second end (116).

10. The drug delivery graft according to claim 9, wherein the diameter of the delivery line (130) proximate the second end (116) is larger than the diameter of the delivery line (130) proximate the first end (114).

11. The drug delivery graft according to any one of claims 1-10, wherein one or both of the graft body (110) and the delivery line (130) is formed of an impermeable material.

12. The drug delivery graft according to any one of claims 1-10, wherein one or both of the first end (114) or the second end (116) are trimmable from an original length to a second selected length, shorter than the original length.

## Patentansprüche

1. Transplantat zur Arzneimittelabgabe (199), umfassend:
einen Transplantatkörper (110), der ein Transplantat-Lumen (112) definiert, das sich zwischen einem ersten Ende (114) und einem zweiten Ende (116) erstreckt;
einen implantierbaren Zugangsport (120);
eine Abgabeleitung (130), die mit dem Transplantatkörper (110) gekoppelt ist und ein Abgabeleitungs-Lumen (132) in Fluidverbindung mit dem implantierbaren Zugangsport (120) definiert;
wobei das Transplantat zur Arzneimittelabgabe **dadurch gekennzeichnet ist, dass** es Folgendes umfasst
eine Vielzahl von Kanälen (140), die sich durch eine Wand des Transplantatkörpers (110) hindurch erstreckt und zwischen dem Transplantat-Lumen (112) und dem Abgabeleitungs-Lumen (132) in Verbindung steht, wobei die Kanäle (140) ein Kanal-Lumen definieren, das Fluidverbindung zwischen dem Transplantat-Lumen (112) und dem Abgabeleitungs-Lumen (132) bereitstellt.

2. Transplantat zur Arzneimittelabgabe nach Anspruch 1, wobei ein Abschnitt der Abgabeleitung (130) mit einer Außenoberfläche des Transplantatkörpers (110) gekoppelt oder innerhalb der Wand des Transplantatkörpers (110) eingebettet oder teilweise innerhalb der Wand des Transplantatkörpers (110) eingebettet ist.

3. Transplantat zur Arzneimittelabgabe nach einem der Ansprüche 1-2, wobei sich die Abgabeleitung (130) spiralförmig um den Transplantatkörper (110) herum erstreckt.

4. Transplantat zur Arzneimittelabgabe nach einem der Ansprüche 1-2, wobei sich die Abgabeleitung (130) seitlich um den Transplantatkörper (110) herum oder in Längsrichtung entlang des Transplantatkörpers (110) erstreckt oder sich von einem Punkt auf dem Transplantatkörper erstreckt und sich in einem radialen Muster über die Oberfläche des Transplantatkörpers (110) erstreckt.

5. Transplantat zur Arzneimittelabgabe nach einem der Ansprüche 1-4, wobei die Vielzahl von Kanälen (140) im gleichen Abstand durch den Transplantatkörper (110) beabstandet ist.

6. Transplantat zur Arzneimittelabgabe nach einem der Ansprüche 1-5, wobei die Vielzahl von Kanälen (140) einen gleichen Lumendurchmesser aufweist.

7. Transplantat zur Arzneimittelabgabe nach einem der Ansprüche 1-4, wobei ein Abstand zwischen einem ersten Kanal (140A) und einem zweiten Kanal (140B) der Vielzahl von Kanälen, die nahe dem ersten Ende (114) angeordnet ist, größer ist als ein Abstand zwischen einem dritten Kanal (140C) und einem vierten Kanal (MOD) der Vielzahl von Kanälen, die nahe dem zweiten Ende (116) angeordnet ist.

8. Transplantat zur Arzneimittelabgabe nach Anspruch 7, wobei ein Durchmesser des ersten Kanals (140A) der Vielzahl von Kanälen, die nahe dem ersten Ende (114) angeordnet ist, kleiner ist als ein Durchmesser des vierten Kanals (MOD) der Vielzahl von Kanälen, die nahe dem zweiten Ende (116) angeordnet ist.

9. Transplantat zur Arzneimittelabgabe nach einem der Ansprüche 1-8, wobei der implantierbare Zugangsport (120) mit der Abgabeleitung (130) nahe dem zweiten Ende (116) gekoppelt ist.

10. Transplantat zur Arzneimittelabgabe nach Anspruch 9, wobei der Durchmesser der Abgabeleitung (130) nahe dem zweiten Ende (116) größer ist als der Durchmesser der Abgabeleitung (130) nahe dem ersten Ende (114).

11. Transplantat zur Arzneimittelabgabe nach einem der Ansprüche 1-10, wobei eines oder beide von dem Transplantatkörper (110) und der Abgabeleitung (130) aus einem undurchlässigen Material gebildet sind.

12. Transplantat zur Arzneimittelabgabe nach einem der Ansprüche 1-10, wobei eines oder beide von dem ersten Ende (114) oder dem zweiten Ende (116) von einer ursprünglichen Länge auf eine zweite ausgewählte Länge, die kürzer als die ursprüngliche Länge ist, zuschneidbar sind.

## Revendications

1. Greffon (199) d'administration de médicament, comprenant :
un corps (110) de greffon, définissant une lumière (112) de greffon s'étendant entre une première extrémité (114) et une deuxième extrémité (116) ;
une chambre implantable (120) ;
une conduite d'administration (130), reliée au corps (110) de greffon et définissant une lumière (132) de conduite d'administration en communication fluidique avec la chambre implantable (120) ;
le greffon d'administration de médicament étant **caractérisé en ce qu'**il comprend
une pluralité de canaux (140), s'étendant à travers une paroi du corps (110) de greffon et communiquant entre la lumière (112) de greffon et la lumière (132) de conduite d'administration, lesdits canaux (140) définissant une lumière du canal fournissant une communication fluidique entre la lumière (112) de greffon et la lumière (132) de conduite d'administration.

2. Greffon d'administration de médicament selon la revendication 1, dans lequel une portion de la conduite d'administration (130) est reliée à une surface externe du corps (110) de greffon, ou incorporée dans la paroi du corps (110) de greffon, ou partiellement incorporée dans la paroi du corps (110) de greffon.

3. Greffon d'administration de médicament selon l'une quelconque des revendications 1 à 2, dans lequel la conduite d'administration (130) s'étend de manière hélicoïdale autour du corps (110) de greffon.

4. Greffon d'administration de médicament selon l'une quelconque des revendications 1 à 2, dans lequel la conduite d'administration (130) s'étend latéralement autour du corps (110) de greffon ou longitudinalement le long du corps (110) de greffon, ou s'étend à partir d'un point sur le corps de greffon et s'étend selon un agencement radial sur la surface du corps (110) de greffon.

5. Greffon d'administration de médicament selon l'une quelconque des revendications 1 à 4, dans lequel les canaux de la pluralité de canaux (140) sont espacés à égale distance dans l'ensemble du corps (110) de greffon.

6. Greffon d'administration de médicament selon l'une quelconque des revendications 1 à 5, dans lequel les canaux de la pluralité de canaux (140) présentent un diamètre de lumière égal.

7. Greffon d'administration de médicament selon l'une quelconque des revendications 1 à 4, dans lequel une distance entre un premier canal (140A) et un second canal (140B) de la pluralité de canaux disposés à proximité de la première extrémité (114), est plus grande qu'une distance entre un troisième canal (140C) et un quatrième canal (MOD) de la pluralité de canaux disposés à proximité de la deuxième extrémité (116).

8. Greffon d'administration de médicament selon la revendication 7, dans lequel un diamètre du premier canal (140A) de la pluralité de canaux disposés à proximité de la première extrémité (114) est plus petit qu'un diamètre du quatrième canal (MOD) de la pluralité de canaux disposés à proximité de la deuxième extrémité (116).

9. Greffon d'administration de médicament selon l'une quelconque des revendications 1 à 8, dans lequel la chambre implantable (120) est reliée à la conduite d'administration (130) à proximité de la deuxième extrémité (116).

10. Greffon d'administration de médicament selon la revendication 9, dans lequel le diamètre de la conduite d'administration (130) à proximité de la deuxième extrémité (116) est plus grand que le diamètre de la conduite d'administration (130) à proximité de la première extrémité (114).

11. Greffon d'administration de médicament selon l'une quelconque des revendications 1 à 10, dans lequel un, ou les deux, parmi le corps (110) de greffon et la conduite d'administration (130) est formé en un matériau imperméable.

12. Greffon d'administration de médicament selon l'une quelconque des revendications 1 à 10, dans lequel une, ou les deux, parmi la première extrémité (114) et la deuxième extrémité (116), sont aptes à être raccourcies par coupe d'une longueur d'origine à une deuxième longueur sélectionnée, plus courte que la longueur d'origine.
